# EUROPEAN PATENT APPLICATION

(11) **EP 1 081 496 A1**
(43) Date of publication of application: **07.03.2001**
(21) Application number: 00115833.6
(22) Date of filing: 24.07.2000
(51) Int. Cl.: G01N 33/569, G01N 33/543, G01N 33/535, C12Q 1/70

(54) **Flow-through assay for visually detecting the presence of influenza A and B**

(30) Priority: 31.08.1999 US 387609
(71) Applicant: Becton, Dickinson and Company, Franklin Lakes, New Jersey 07417-1880 (US)
(72) Inventor: Milunic, David, Stillwater, Minnesotta 55082 (US); Lovell, Stephen J., Lutherville, Maryland 21093 (US)
(74) Representative: von Kreisler, Alek, Dipl.-Chem.

(57) **Abstract**

The present invention relates to a diagnostic test for detecting a human influenza A and B antigen in a clinical specimen. The antigen is detected by determining the presence of antibodies specific for influenza A or B. Antibody A and B are conjugated to a characteristic enzyme and, if antigen is present, the enzyme reacts with one or more substrates to produce a detectable reaction product.

## Description

### Field of the Invention

The present invention relates to a diagnostic test for detecting a human influenza A and B antigen in a clinical specimen. The antigen is detected by determining the presence of antibodies specific for influenza A or B. Antibody A and B are conjugated to a characteristic enzyme and, if antigen is present, the enzyme reacts with one or more substrates to produce a detectable reaction product.

### Background of the Invention

The rapid diagnosis of viral infections is becoming an integral part of good medical practice. Some viruses have definable antigens against which antibodies can be produced. These antibodies and antigens, i.e. immunoreactants, are capable of binding with one another, thereby creating a highly specific reaction mechanism which can be used in vitro to determine the presence or concentration of that particular antigen in a biological sample. Therefore, immunoassays have been widely used for the measurement of the antigen and the subsequent determination of the presence or absence of a virion.

There are several known immunoassay methods using immunoreactants, wherein at least one of the immunoreactants is labeled with a detectable component so as to be analytically identifiable. For example, the "sandwich" or "two-site" technique may involve the formation of a ternary complex between an antigen and two antibodies. A convenient method of detecting complex formation in such a technique is to provide one labeled antibody and an unlabeled antibody bound to a solid phase support such that the complex can readily be isolated. In this example, the amount of labeled antibody associated with the solid phase is directly proportional to the amount of analyte in the test sample.

An alternative technique is the "competitive" assay. In one example of a competitive assay, the capture mechanism again may use an antibody attached to an insoluble solid phase, but a labeled analyte (rather than a labeled antibody) competes with the analyte present in the test sample for binding to the immobilized antibody. Similarly, an immobilized analyte can compete with the analyte of interest for a labeled antibody. In these competitive assays, the quantity of captured labeled reagent is inversely proportional to the amount of analyte present in the sample.

There are a number of assay devices and procedures wherein the presence of an analyte is indicated by the analyte's binding to a labeled reagent or a complementary binding member that is immobilized on a solid phase such as a dipstick, teststrip, flow-through pad, paper, fiber matrix or other solid phase material. Such a specific binding reaction results in a distribution of the labeled reagent between that which is immobilized upon the solid phase and that which remains free. Typically, the presence or amount of analyte in a test sample is indicated by the extent to which the labeled reagent becomes immobilized upon the solid phase.

The use of porous teststrips in the performance of specific binding assays is also well-known. In a sandwich assay procedure, a test sample is applied to one portion of the teststrip and is allowed to migrate through the strip material by means of capillary action. The analyte to be detected or measured passes through the teststrip material and is transported into a detection zone on the teststrip wherein the analyte-specific binding member is immobilized. The extent to which the analyte becomes bound in the detection zone is then determined.

A variety of binding methods have been used to remove an analyte from a test solution. U.S. Patent No. 4,020,151 describes a solid-phase assay for the quantitation of antigens or antibodies in a test sample. The sample antigen or antibody is adsorbed directly onto a solid support surface, such as anion exchange resin, and the support is then exposed to a labeled specific binding member that is immunologically reactive with the sample antigen or antibody.

Other assay methods involve the use of auxiliary specific binding members. U.S. Patent No. 4,624,930 describes a process for determining the presence of a polyvalent antigen by incubating the antigen with three receptors; a first and a third receptor which bind to the antigen and a second receptor, bound to a solid support, which specifically binds to the first receptor.

Examples of devices utilizing some of the above principles are described in U.S. Pat. Nos. 5,866,322, 5,663,055, 4,094,647, 4,235,601, 4,361,537, 4,366,241, 4,740,468, 4,879,215, 4,956,275, 4,059,407, 3,802,842, 3,915,639, 4,689,309, 4,168,146, 4,435,505, 4,594,327, 4,757,004, 4,956,302, 4,020,151, 4,145,406, 4,211,763, 4,362,697, 4,517,288, 4,624,930, 4,343,896, 4,880,751, 4,298,685, 4,935,147, 4,948,726, 4,959,303, 4,990,442, 4,839,231, 4,780,409, and 4,530,900, which are incorporated herein by reference.

As will be appreciated by the foregoing discussion, there is significant activity in the field of diagnostics. There is an ever-growing demand for techniques and devices that are relatively simple and inexpensive to use.

### Summary of the Invention

The present invention is directed to a method for diagnosing human influenza A or B comprising the steps of a) providing a test sample suspected of containing influenza A or influenza B virus; b) contacting the test sample with a solid phase to which viral antigen binds; c) contacting the solid phase with anti-influenza A antibodies coupled to a first enzyme and anti-influenza B antibodies coupled to a second enzyme; d) contacting the solid phase with substrates for the first and second enzymes; and e) determining the presence or amount of viral antigen by visibly detecting a colored precipitate deposited on the solid phase by action of at least one of the enzymes.

### Brief Description of the Drawings

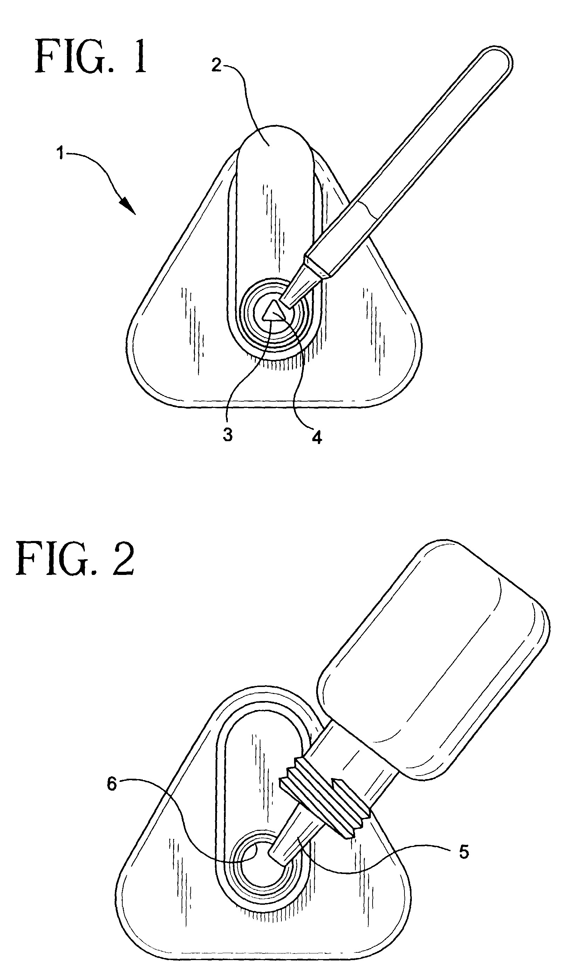

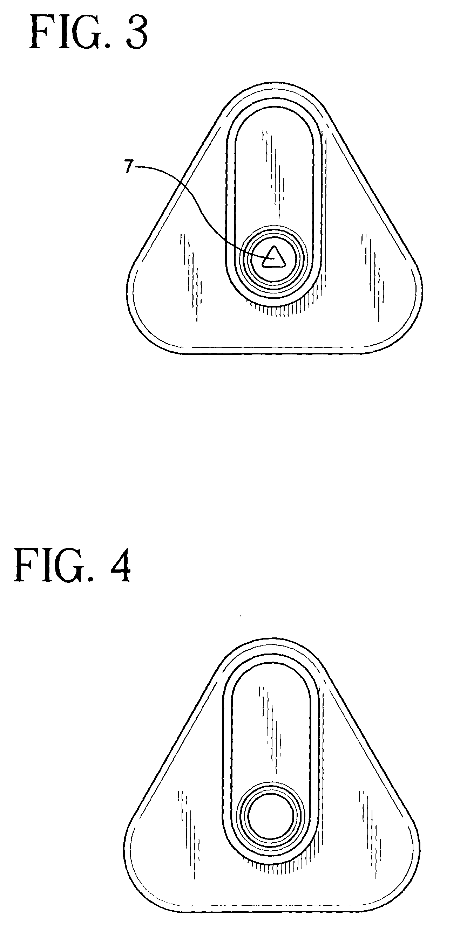

FIGS. 1 through 4 generally illustrate an assay according to the present invention.
FIG. 1 shows a sample being applied to a flow controller of a *Direct*igen® (Becton Dickinson) influenza detection device.
FIG. 2 shows a wash solution being applied to the flow controller.
FIG. 3 shows a positive test result indicating the presence of influenza A antigen.
FIG. 4 shows a negative test result indicating no presence of influenza A antigen.

### Detailed Description of the Invention

The present invention may be used to detect the presence of human influenza A and B in a clinical specimen. The clinical samples that are tested in the invention typically will be nasal, pharyngeal, nasopharyngeal or respiratory secretions collected as wash, expectorate, aspirate or swab specimens.

Reagents which can be utilized in the present invention can be prepared as described in the *Direct*igen® Flu A products insert, incorporated herein by reference, except that in a preferred embodiment, thimerasol can be substituted for sodium azide.

An example of the kinds of reagents which can be used in a preferred embodiment of the invention, and how they can be used, is set forth below as follows.
Reagent A (extraction reagent) -- as in *Direct*igen® Flu kit, but 0.2% thimerasol can be used in place of sodium azide.
   Extraction, 1.6% mucolytic agent and 6.4% detergent, with 0.2% thimerasol (preservative).
Reagent 1 -- no change (no azide)
   Wash, 150 mM citric acid.
Reagent 2 -- as in *Direct*igen® Flu kit, but 0.2% thimerasol can be used in place of sodium azide.
   Wash, 50 mM Tris and rabbit IgG with 0.2% thimerasol (preservative).
Reagent 3 -- Anti-influenza A monoclonal antibodies (2) coupled to alkaline phosphatase and anti-influenza B monoclonal antibody (1) coupled to horseradish peroxidase (Pierce 31497) in Tris buffered diluent.
Reagent 4 -- as in *Direct*igen® Flu kit but 0.2% thimerasol can be used in place of sodium azide.
   Wash, 5% butanol, 2M urea and 100 mM Hepes with 0.2% thimerasol (preservative).
Reagent 5 -- as in *Direct*igen® Flu kit but 0.2% thimerasol can be used in place of sodium azide.
   Wash, 50 mM Tris and 150 mM NaCl with 0.2% thimerasol (preservative).
Reagent 6 -- Alkaline phosphatase substrate (Moss, Inc. Product No. INTH - 1000)
Reagent 7 -- KPL HRP substrate (50-77-00) TMB membrane peroxidase substrate system.

In a preferred embodiment, antigens which can be utilized in the present invention are: for Flu B, B Lee 40 strain; and for Flu A, Flu A virus of the subtype H1N1.

Solid phase devices utilized in the present invention can be selected, for example, from *Direct*igen® test kits.

It is well-known to those skilled in the art that anti-influenza A and anti-influenza B antibodies may be coupled with enzymes. Such enzymes include, for example, peroxidase, urease, galactosidase, glucose oxidase, alkaline phosphatase and beta-glucuronidase.

Referring to the FIGURES, the assay of the present invention is performed according to the following scheme:
1. Once the sample is extracted, it is applied to the flow controller (2) of a *Direct*igen® influenza detection device (1). The flow controller (2) is provided with a triangular opening(3) and the sample flows through the opening (3) into a nylon membrane (4). The nylon membrane (4) is supported on an absorbent material, thus, the sample, which flows through the nylon membrane (4) permitting antigen to bind non-specifically to the membrane (4) in the shape of a triangle, also flows into the absorbent material.
2. The flow controller (2) with its triangular opening (3), is removed from the device (1). The device (1) with nylon membrane (4) is provided with a circular opening(6).
3. A wash solution is then applied to the nylon membrane (4) of the device (1).
4. A tris-buffered diluent containing anti-influenza antibodies conjugated to enzymes is then applied. If the influenza antigen is present, the antibodies will bind thereto in the shape of a triangle.
5. Wash solutions are applied and then a substrate appropriate for the enzymes is applied. If influenza antigen is present, a colored triangle (7) will be visible.

Preferably, the assay will detect influenza A and influenza B by a differentiating test. At step 5 above, the solution is provided with influenza A and influenza B antibodies conjugated to different enzymes. The presence of a specific antigen would be detected by the color of the triangle produced.

The differentiating test might utilize alkaline phosphatase conjugated to influenza A antibody and horseradish peroxidase conjugated to influenza B antibody. At step 5 above, a substrate for alkaline phosphatase would be added and if influenza A antigen is present, an orange triangle will result. In a preferred embodiment the substrate mixture for alkaline phosphatase can be bromochloroindotyl phosphate and p-iodonitrotetrazolium. If no influenza A antigen is present, the nylon membrane will remain uncolored. A substrate for horseradish peroxidase would then be added. In a preferred embodiment, the substrate mixture for horseradish peroxidase can be 3, 3', 5, 5'-tetramethyl-benzidine and hydrogen peroxide. If influenza B antigen is present, a blue triangle will result. If no influenza B antigen is present, the nylon membrane will remain uncolored. Most preferably, influenza A and influenza B substrates are combined as a single reagent thereby avoiding the need for sequential addition of reagents.

### Example 1

This example illustrates the differentiated method of detecting the presence of human influenza A or human influenza B according to the assay of the present invention with the following steps:
1. Devices from a *Direct*igen® Flu kit were used in the experiments.
2. Antigen preparations consisted of a preparation of a Flu A virus of the subtype H1N1 and a preparation of Flu B Lee 40.
   The negative control was detergent-treated egg fluid (non-infected) with 0.2% sodium azide.
3. Reagent solutions were prepared as for the *Direct*igen® Flu A kit except that sodium azide was replaced with thimerasol. The solutions were dispensed into dropper vials in the volumes detailed in the Product Insert from the *Direct*igen® Flu A kit.
4. The negative control, Flu A antigen, and Flu B antigen were mixed separately with extraction reagent A as described in the *Direct*igen® Product Insert; four (4) drops each of antigen and negative control were dispensed into a tube and followed by eight (8) drops of reagent A.
5. Extracted Flu A antigen was dispensed dropwise into the device and allowed to absorb completely.
6. Reagent 1 was added to the device until the well was filled and allowed to absorb completely.
7. The flow controller was removed and Reagent 2 [four (4) drops] was applied to the device membrane. The reagent was allowed to absorb completely.
8. Four (4) drops of Reagent 3 (anti-Flu A/alkaline phosphatase and anti-Flu B/peroxidase) were dispensed onto the membrane and the device was allowed to stand for two (2) minutes after the reagent had absorbed.
9. Reagent 4 was dispensed until the device well was filled and allowed to absorb completely.
10. Reagent 5 [four (4) drops] was dispensed onto the well and allowed to absorb.
11. Reagent 6 [four (4) drops], the substrate for alkaline phosphatase, were dispensed onto the membrane and the device allowed to stand for five (5) minutes. An intense orange triangle was observed.
12. Reagent 7 [four (4) drops], the peroxidase substrate was dispensed, and the device allowed to stand for five (5) minutes. There was no change in the color of the triangle.
13. Reagent 5 [four (4) drops] was dispensed onto the membrane and allowed to absorb completely.

### Example 2

The experiment was repeated as in Example 1 (with steps 1-13), but with Flu B antigen utilized in place of Flu A antigen (at step 5). In this case, no triangle was observed at step 11. A dark blue triangle was observed at step 12.

The experiment was repeated with negative control solution at step 5. In this case, no triangle was observed at step 11 or step 12.

While this invention is satisfied by embodiments in many different forms, there is described in detail preferred embodiments of the invention. It is to be understood that the present disclosure is to be considered exemplary of the principles herein, it is not intended to limit the invention.

## Claims

1. A method for diagnosing human influenza A or B comprising the steps of a) providing a test sample suspected of containing influenza A or influenza B virus; b) contacting the solid sample with a solid phase to which viral antigen binds; c) contacting the solid phase with anti-influenza A antibodies coupled to a first enzyme and anti-influenza B antibodies coupled to a second enzyme; d) contacting the solid phase with substrates for the first and second enzymes; and e) determining the presence or amount of viral antigen by visibly detecting a colored precipitate deposited on the solid phase by action of at least one of the first and second enzymes.

2. The method according to claim 1 wherein the first enzyme is selected from the group consisting of peroxidase, urease, galactosidase, glucose oxidase, alkaline phosphatase, and beta-glucuronidase, and wherein the second enzyme is selected from the group consisting of peroxidase, urease, galactosidase, glucose oxidase, alkaline phosphatase, and beta-glucuronidase.

3. The method according to claim 1 wherein the solid phase is a device provided in a *Direct*igen® influenza kit.

4. The method according to claim 1 wherein said viral antigen is influenza A antigen or influenza B antigen.

5. The method according to claim 4 wherein the influenza B antigen is B Lee 40 strain.

6. The method according to claim 4 wherein the influenza A antigen is a Flu A virus of the subtype H1N1.

7. The method according to claim 1 wherein influenza A antibodies are conjugated to alkaline phosphatase.

8. The method according to claim 1 wherein the influenza B antibodies are conjugated to horseradish peroxidase.

9. The method according to claim 1 wherein influenza A antibodies are conjugated to beta-glucuronidase.

10. The method according to claim 1 wherein influenza B antibodies are conjugated to beta-glucuronidase.
